# EUROPEAN PATENT SPECIFICATION

(11) **EP 1 088 811 B1**
(45) Date of publication and mention of the grant of the patent: **20.11.2002**
(21) Application number: 99118634.7
(22) Date of filing: 21.09.1999
(51) Int. Cl.: C07C 45/72, C07C 45/75, C07C 45/74, C07C 47/19, C07C 45/80, C07C 29/141, C07C 31/20

(54) **A cross-aldol condensation for hydroxypivaldehyde**
Cross-Aldolkondensation für Hydroxypivaldehyd
Condensation cross-aldolique pour l'hydroxypivaldéhyde

(43) Date of publication of application: 04.04.2001
(73) Proprietor: SAUDI BASIC INDUSTRIES CORPORATION (SABIC), Riyadh 11422 (SA)
(72) Inventor: Mahmud, Meftahuddin, Dr., c/o Mr. Ali Awwad, P.O. Box 42503, 11551 Riyadh (SA); Vishwakarma, Lal C.,Dr., c/o Mr. Ali Awwad, P.O. Box 42503, 11551 Riyadh (SA); Faizi, Ahmad Kamal, c/o Mr. Ali Awwad, P.O. Box 42503, 11551 Riyadh (SA)
(74) Representative: Goddar, Heinz J., Dr.

(56) References cited:
- WO-A-98/29374
- US-A- 3 432 557
- US-A- 4 215 076
- CHEMICAL ABSTRACTS, vol. 106, no. 1, 5 January 1987 (1987-01-05) Columbus, Ohio, US; abstract no. 004499, JIA Y ET AL: "Aldol condensation of aldehydes and butanone with the phase-transfer catalyst benzyltriethylammonium chloride" XP002129892 & FUDAN XUEBAO, ZIRAN KEXUEBAN (FHPTAY,04277104);1986; VOL.25 (1); PP.57-64, Fudan Univ.;Shanghai; Peop. Rep. China (CN)

## Description

### BACKGROUND OF THE INVENTION

### Field of the Invention

This invention relates generally to a novel process for aldol condensation of aldehydes, and particularly to a process for forming desired aldols by the reaction of specified aldehydes in the presence of basic phase-transfer catalyst. More particularly, this invention relates to using tetra alkyl ammonium or phosphonium hydroxides as phase-transfer catalyst in cross-aldol condensation.

### Description of the Prior Art

The reaction of aldehydes in the so-called "aldol condensation" reaction is well known. In this reaction, a nucleophilic carbanion, produced, for example, by the action of a strong base or alkali ion on aldehyde having an α-hydrogen atom, attacks the carbonyl carbon atom of another aldehyde molecule. If the second aldehyde is different from the first one, the process is called "cross-aldol" condensation. The aldehyde-alcohol (aldol) produced by the reaction may be recovered, or it may be treated directly in the reaction system or reaction product admixture to form a desired product. In many instances, if the starting aldehyde had more than one α-hydrogen atom, the aldol de-hydrates spontaneously or promptly in the process admixture or environment, or is easily dehydrated, forming an unsaturated aldehyde, which can be recovered or treated further (US-A-3 432 557).

Hydroxypivaldehyde (HPA), a cross-aldolization product, serves as an important intermediate in the manufacture of neopentyl glycol. Neopentyl glycol, in turn, has been widely used as an intermediate for the synthesis of saturated and unsaturated polyesters and in manufacture of synthetic resin, polyurethanes, synthetic lubricants, polymers and plasticizers.

Traditionally hydroxypivaldehyde has been obtained by cross-aldolization of isobutyraldehyde with formaldehyde using triethyl amine or alkali metal hydroxides or carbonates or alkaline earth metal hydroxides (hydrated) as a base catalyst. Such publications are referenced in this application. These references describe the state of the art to which this invention pertains, and incorporated herein by reference.

US patent 2,818,443 has disclosed a reaction of formaldehyde and an aliphatic aldehyde in the presence of anion exchange polymeric resin catalyst to form hydroxyaldehyde. DE patent 26 53 096 proposes the use of cation exchange polymeric resins as a catalyst for the aldolization reaction. WO 98/29374 (PCT/F197/00835) describes the use of weak base anion exchange polymeric resins as an aldolization catalyst. However, these polymeric resin catalysts in these patents have not been disclosed to involve a phase-transfer mechanism in the aldolization chemistry, handling of these polymeric resins to the precise molar ratio is difficult, require long residence times usually 3 to 10 hours, and more importantly, with these catalysts the maximum conversion of isobutyraldehyde has been up to 86% only whereas nearly 100% conversion is desirable in the high volume manufacturing process.

U.S. Patent Nos. 3,808,280; 4,855,515; 5,185,478; 5,146,012; 5,144,088; and 5,072,058; and WO 98/17614 (PCT/KR97/00199); and EP 484800 teach the use of tertiary alkylamine, such as triethyl amine, as the base catalyst for the cross-aldolization of isobutyraldehyde with formaldehyde or paraformaldehyde. However, elevated temperature, and longer reaction time are usually required. Also, occasionally the selectivity remains moderate.

Solid base catalysts also have been employed in the cross-aldolization reaction. For example, WO 97/35825 (PCT/US97/04926), discloses the use of hydrated magnesium oxide. However, this process suffers from the disadvantage that isobutyraldehyde is taken in large excess, at least 3 mole equivalent, it needs longer time, and at least two side products are formed. U.S. Pat. No, 2,811,562 has disclosed the use of alkali metal carbonate such as potassium carbonate as a base catalyst. The disadvantage in this process is that it requires an elevated temperature such as 60-75°C, and long reaction time usually 2 - 4 hours. U.S. Pat. No. 3,920,760 has disclosed the use of sodium carbonate base but do not demonstrate any better advantage over potassium carbonate.

U.S. Pat. No. 5,841,002 discloses the use of sodium hydroxide base catalyst for cross-aldolization of isobutyraldehyde with formaldehyde. However, recycling of base involves quite elaborate electrolytic process. Moreover, the use of alkali metal hydroxide base leads to crossed Canizzaro and Tischenko reaction side products even if the reaction temperature rises slightly.

US-A-4 215 076 discloses aldole reactions of aliphatic aldehydes or ketones in the presence of an aqueous base as catalyst and a neutral or cationic surfactant as a cocatalyst. Tetraalkylammonium halides have been disclosed as cocatalysts.

Chem. Abs. vol. 106, nº1 (1987) 4499Z discloses the aldol condensation of aldehydes and butanone with the phase-transfer catalyst benzyltriethylammonium chloride.

Therefore, one of the objectives of the present invention is to overcome the difficulties and disadvantages encountered in the prior art by providing a phase-transfer catalyzed novel process for the synthesis of hydroxypivaldehyde.

### Summary of the Invention

This invention also relates to an aldehyde condensation process comprising contacting or combining a first aldehyde selected from aldehydes containing an α-hydrogen atom having the formula R₁CHO, wherein R₁ is selected from alkyl having one to twelve carbon atoms, and aralkyl having seven to fourteen carbon atoms, with a second different aldehyde selected from aldehydes having the formula R₂CHO, wherein R₂ is selected from H, alkyl having one to twelve carbon atoms, and aralkyl having seven to fourteen carbon atoms, with a catalytic amount of only a phase-transfer catalyst of Formula (I), where Y is a hydroxide anion in this invention. 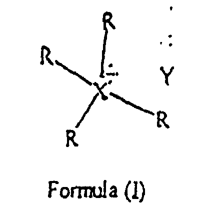 [Where, R = same or different alkyl or aralkyl group, X = nitrogen or phosphorus atom, Y = OH, counter anion.]

The product mixture will comprise or contain an aldol of said aldehydes, the unsaturated aldehyde derived by dehydration of said aldol, or mixtures thereof. Accordingly, the reaction product mixtures of the invention will comprise or contain one of (A) the aldol of the aldehyde condensed, or of the aldehydes cross condensed; (B) the unsaturated aldehyde derived by dehydration of said aldol; or (C) a mixture of (A) and (B).

As used herein, the expression "the unsaturated aldehyde derived by dehydration of said aldol" refers to the α,β-olefinic aldehyde resulting from the decomposition of the aldol produced by the condensation, which decomposition also results in or is characterized by the formation of water as a product.

Further, according to the present invention, any stable aldol formed may be separated from the reaction mixture produced, and treated as desired, e. g., hydrogenated to form a hydrogenated mixture containing a di-alcohol therein.

The invention is particularly useful for the condensation of n-butyraldehyde and the cross condensations of n-butyraldehyde, or isobutyraldehyde, with formaldehyde.

### Advantageous Effect of the Invention

According to this invention, no more than 4 mole % equivalent of phase-transfer catalyst is needed in this reaction. No other (or additional) base is required by virtue of this phase-transfer catalyst containing hydroxide counter anion (a basic entity necessary to initiate the first step in the reaction by de-protonation of the α-hydrogen atom of isobutyraldehyde). The catalyst can be easily recovered by extraction with a suitable solvent. This reagent provides 100% selectivity of the product hydroxypivaldehyde (including 2-5% of NPG formed). It should be noted that hydroxypivaldehyde being the precursor intermediate for neopentyl glycol, to our advantage, neopentyl glycol is already being formed from it in small amount.

### Detailed Description of the Invention

The following is the basic reaction of the present invention:

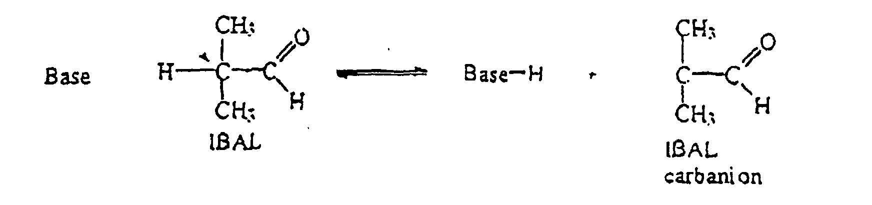

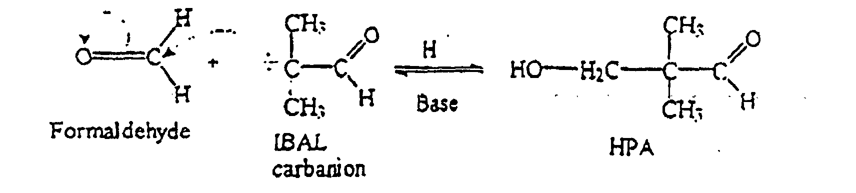

This reaction represents cross-aldol condensation between two different aldehydes, namely isobutyraldehyde having an α-hydrogen atom and formaldehyde having no α-hydrogen atom, for the synthesis of aldehyde-alcohol, namely hydroxypivaldehyde (HPA). The expression "formaldehyde" comprises both conventional formaldehyde obtained as an aqueous solution and anhydrous forms of formaldehyde, such as paraformaldehyde and trioxane. Commercially available aqueous formaldehyde usually also contain relatively small amounts of methanol.

The problem at hand in this invention is the following. Reaction between two substances located in different phases of a mixture is often inhibited because of the inability of reagents to come together. Isobutyraldehyde is a liquid material that makes the water insoluble organic phase. On the other hand, formaldehyde solution makes the aqueous phase because it comes as 37 - 40% aqueous solution. In the aldolization process, these two reacting components create a non-homogeneous two phase reaction medium. Therefore, interaction of the two reacting components is not sufficient enough for smooth cross-aldolization leading to the product hydroxypivaldehyde. Thus, the equilibrium in the first step of the reaction mechanism tries to be more to the backward (i.e., to the starting isobutyraldehyde).

One way to solve this problem is to make the reaction mixture homogeneous by using a solvent such as methanol or ethanol. However, by doing this we will decrease the concentration of the reacting components, thereby decreasing the reaction rate.

The another but better way to solve this problem, according to the present invention, is to make use of an appropriate basic phase-transfer catalyst (PTC). Reaction is brought about by the use of small quantities of an agent, which transfers one reactant across the interface into the other phase, so that reaction can proceed. Thus, phase-transfer catalysts are defined as compounds whose addition to a two-phase organic-water system helps transfer a water soluble reactant across the interface to the organic phase where a homogeneous reaction can occur. This can also-help transfer a water insoluble reactant across the interface to the aqueous phase where a homogeneous reaction can occur.

The phase-transfer agent is not consumed but performs the transport function repeatedly. Organic-soluble quaternary ammonium or phosphonium cations have been found to be excellent agents for the transport of anions from aqueous phase to an organic phase.

We have chosen a specific phase-transfer catalyst, namely benzyltrimethyl or benzyltriethyl ammonium hydroxide which is also known as Triton B (methyl version) (Fluka Catalog # 13991). This comes as a 40% solution in water. Its chemical formula is given below: 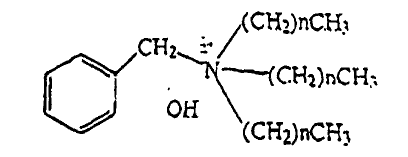 where, n = 0 or 1

The hydroxide counter anion acts as a base entity to de-protonate α-hydrogen of the isobutyraldehyde molecule, whereas the quaternary ammonium cation part assists in the phase-transfer mechanism.

To achieve similar results from anhydrous formaldehyde, such as paraformaldehyde or trioxane, preferably some amount of water could be added to generate a binary phase reaction medium.

There are a few relevant references in the literature on phase-transfer catalyzed aldol condensation with aromatic aldehydes but none found with aliphatic aldehydes Products are frequently olefinic compounds as a result of dehydration favored by their thermodynamic stability. The following examples of aromatic aldehydes may be cited to substantiate the present invention. 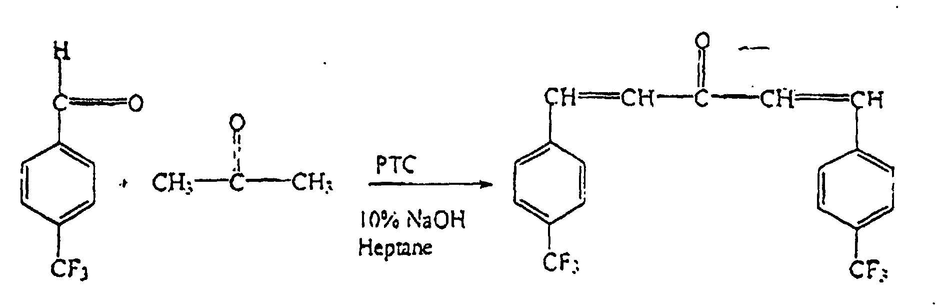 Cortese, N.: Gastrock, W.: (American Cyanamid), U.S. Patent 4,521,629 (1985) 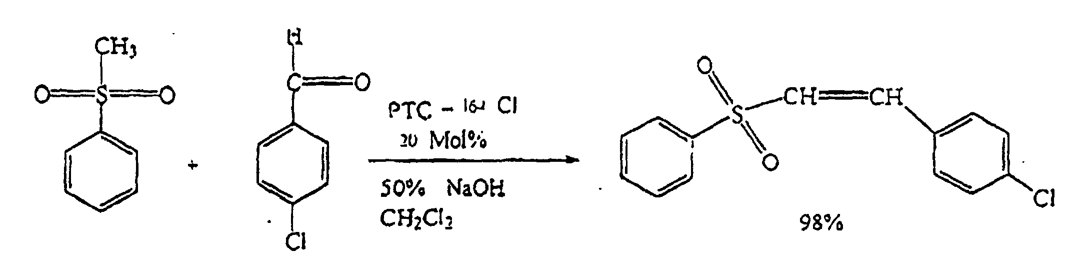 Cardillo, G. Savoia, D: Umani-Ronchi, A.: *Synthesis*, 1975, 453

These references, however, are far from the present invention because they are not representing condensation reactions between two same kind of aldehydes or two different kind of aldehydes.

Thus, our invention seems to be by far the first example of phase-transfer catalyzed aldol condensation involving two different kind of aliphatic aldehydes, thereby making an excellent representation of cross-aldol condensation.

The concept of basic phase-transfer catalysis is easily extended to aldehydes in which at least one of the aldehydes should contain an α-hydrogen atom. The said aldehyde may have a general formula of R₁CHO, where R₁ is selected from alkyl having 1-12 carbon atoms, cycloalkyl, aryl, and aralkyl having 7-14 carbon atoms. The aldolization reaction forms a reaction product mixture comprising or containing an aldol of said aldehyde, an unsaturated aldehyde derived by dehydration of said aldol, or mixtures thereof. Meaning of unsaturated aldehyde has been defined previously.

The present invention preferably relates to an aldolization reaction of aldehydes containing a mixture of an aldehyde containing α-hydrogen atom defined above and a second different aldehyde having a general formula of R₂CHO, where R₂ is selected from H, alkyl having 1-12 carbon atoms, cycloalkyl, aryl and aralkyl having 7-14 carbon atoms, in the presence of catalytic amount of benzyltriethyl ammonium hydroxide.

Suitable starting aldehydes are, for example, formaldehyde, ethanal, propanal, butanal, pentanal, 2-methylpropanal(isobutyraldehyde), 2-methyl butanal, 2-ethylpentanal, 2-ethylhexanal, 2-isopropylbutanal, 2-phenylpropanal, 2-cyclohexylpropanal, 2-phenylbutanal, 2,3-diphenylpropanal, cyclopentylaldehye and cyclohexylaldehye.

While it is not a requirement of the invention that the aldehyde or aldehydes chosen be pure or substantially pure, selectivity to the aldol may be impaired in the reaction in proportion to the amount of impurity or undesired reactant present. Accordingly, some concentration of other compositions may be present in the aldehyde or aldehydes employed so long as the compositions do not interfere significantly with the condensation reaction or with the desired end use of the products of the invention.

The prior art Patent No. WO 9829374 (9 July 1998) is assigned to NESTE OY in Finland. Its title is "Process for the Preparation of Polyvalent Alcohols". The main feature disclosed in the prior art is using polymeric resin-based catalyst for aldolization and claiming several advantages for using such catalysts. These advantages include good yield without notable side reactions, no separation and recycling of catalyst and easy re-generation of catalyst. The prior art does not disclose the mode of action of the catalyst. Weak basic nature of the catalyst would initiate de-protonation of α-hydrogen atom of the isobutyraldehyde molecule in order to proceed to cross-aldolization. However, the prior art does not envision the process as phase-transfer catalyzed. Therefore, our invention is clearly distinct from this prior art.

The distinction and advantages of the present invention over this prior art is further illustrated from Table 1.

**Table 1**

| | Results/Comments | |
|---|---|---|
| | **Patent WO 98/29374 (Neste)** **(Closest prior art)** | **Present Invention** **(Phase-transfer catalysis)** |
| Catalyst type | Polystyrene-DVB based amine or quat. amine bases (Resin) | Simple PTC agent such as benzyltrimethyl ammonium hydroxide |
| Conversion | 95.8% | ∼ 95.17% |
| Selectivity | 98.1% | 100% |
| Catalyst amount | Typically 25-60% by wt. (based on isobutyraldehyde) | ∼ 4% (wt) |
| Reaction time | > 7 hours | 0.5 - 1.0 hour |
| By-products formation | By-product (HPHP)/HPA = 3.5% | By-product (HPHP)/HPA = 0% |
| Characteristics concerns | Not easy to handle in recycling due to swelling | Can be recycled after extraction |

In the present invention, the reaction solvent system may comprise of a combination of miscible and immiscible solvents, preferably of water and an organic solvent, or most preferably of an aqueous solution of first aldehyde and of second aldehyde as neat liquid (if it is not a solid). The reaction temperature may be varied from zero to 30 °C, but the most preferred range is 18 - 25 °C. The reaction time may be varied from 10 - 120 minutes. However, the most preferred reaction time is 30 - 90 minutes.

The present invention is illustrated in greater detail by the following examples, Examples 1 and 2 being comparative examples.

### EXAMPLE 1 (Comparative Example)

### Cross-Aldolization using ammonium hydroxide as the base catalyst at 25 °C:

The following quantities of reactants were taken in this experiment as described in the Table 2.

**Table 2**

| Isobutyraldehyde | 37% HCHO aq.solution | 25% Aqueous NH₄OH |
|---|---|---|
| 3.6g (0.05 Mole), MW 72, | 4.22 g of 37% aq. solution (0.052 Mole, d=1.081; | 0.175 g (0.005 Mole) |
| d=0.7938, 3.9 mL | 0.39 mL MW 30 | |

The two aldehydes were taken together in a reaction flask under argon atmosphere. It was cooled to 25 °C. Aqueous ammonium hydroxide was added dropwise to the stirring reaction mixture. No exotherm was observed. The reaction was run at the same temperature for 2 hours. The GC analysis did not show any significant amount of desired product hydroxypivaldehyde.

### EXAMPLE 2 (Comparative Example)

### Cross-Aldolization using triethylamine as the base catalyst at 25 °C:

The following quantities of reactants were taken in this experiment as described in the Table 3.

**Table 3**

| Isobutyraldehyde | 37% HCHO aq.solution | Triethylamine |
|---|---|---|
| 7.2 g (0.10 Mole), MW | 10.14 g of 37% aq. solution | 0.5 g (0.005 Mole), |
| 72, d=0.7938, 9.1 mL | (0.125 Mole, d=1.081; 0.9.4 mL MW 30 | 0.7 mL, d = 0.726 |

The two aldehydes were taken together in a reaction flask under argon atmosphere. It was cooled to 25 °C. Triethylamine was added dropwise to the stirring reaction mixture. No exotherm was observed. The reaction was run at the same temperature for 1.5 hours. The GC analysis did not show any significant amount of desired product, hydroxypivaldehyde.

### EXAMPLE 3 (Comparative Example)

### Cross-Aldolization using triethylamine as the base catalyst at 65 °C:

The following quantities of reactants were taken in this experiment as described in the Table 4.

**Table 4**

| Isobutyraldehyde | 37% HCHO aq.solution | Triethylamine |
|---|---|---|
| 7.2 g (0.10 Mole), MW | 10.14 g of 37% aq. solution | 0.5 g (0.005 Mole), |
| 72, d=0.7938, 9.1 mL | (0.125 Mole, d=1.081; 0.9.4 mL MW 30 | 0.7 mL, d = 0.726 |

The two aldehydes were taken together in a reaction flask under argon atmosphere. It was cooled to 25 °C. Triethylamine was added dropwise to the stirring reaction mixture. No exotherm was observed. The reaction temperature was raised to 65 °C. The reaction was run at the same temperature for 1.5 hours. The GC analysis showed 53.81% peak area of desired product, hydroxypivaldehyde with about 90% selectivity.

The following example of the present invention (Example 4) provides far superior result, e.g. 82.09% peak area and 100% selectivity, of hydroxypivaldehyde.

### EXAMPLE 4 (Invention Example)

The experiment of the present invention is fully illustrated by the following detailed steps and analytical data for the result.

The apparatus was set up on a water bath (water chiller) using a 4-necked 250 mL round-bottomed flask (all joints S24/40). Mechanical stirring rod was attached in the central neck, a digital thermocouple inlet in the hind side neck, stopper in the front side neck, a dropping funnel in the right side neck. Argon gas inlet/ outlet were attached through a rubber septum, the outlet tube was attached to a mineral oil bubbler. The system was purged with argon gas for 5-10 minutes. A small short stem glass funnel was put in the neck in place of the stopper. Isobutyraldehyde was added followed by formaldehyde solution. It was stirred at 20°C at such a rate that the contents of the flask did not splash out. The funnel was removed. The solution of the phase-transfer catalyst was added dropwise through this neck at such a rate that temperature did not rise beyond 21-22°C. Slow addition of phase-transfer catalyst solution is very critical because of significant exotherm. The addition of phase-transfer catalyst may take as long as 30-35 minutes. In order to control the temperature, it may become necessary to stop the addition temporarily for a few minutes. [*Caution*: Exotherm may be as high as 30-40°C if PTC added all at once]. As indicated by GC analysis, surprisingly about 80% conversion takes place within 5 minutes. Generally reaction gets completed within 60 minutes with over 90% conversion of isobutyraldehyde. The sample aliquots for GC analysis were taken after 30, 60, and 90 minutes. Added 1-2 drops of glacial acetic acid for neutralization of the base before GC injection. (Addition of acetic acid may not be necessary). Generally, the product starts solidifying after 30-40 minutes. At 60 minutes, it may be completely solid and stirring of the reaction mixture becomes inefficient. In this situation, 10 mL of methanol is added through the dropping funnel while raising the temperature to 35°C and stir for another 30 minutes.

The Table 5 summarizes the GC analysis result for the 90 minute injection.

**Table 5**

| GC Analytical Data | | | | |
|---|---|---|---|---|
| **Component** | **Retention Time** **(Minute)** | **% Peak Area** | **%Conversion of IBAL** | **Selectivity of HPA (including NPG)** |
| Isobutyraldehyde | 2.33 | 4.83 | 95.17 | --- |
| Unknown | 2.87 | 2.25 | ---- | ---- |
| HPA (Product) | 6.08 | 82.09 | ---- | 100% |
| NPG(Final) | 8.13 | 4.11 | --- | (included with HPA) |
| Unknown (PTC) | 10.17 | 2.46 | --- | --- |
| Unknown (PTC) | 10.79 | 1.14 | --- | --- |
| Unknown (PTC) | 12.30 | 3.13 | ---- | ---- |

This reaction, including the scale-ups, is well reproducible. The product (hydroxypivaldehyde) was isolated by extracting with a suitable solvent such as ethyl acetate or isobutyl acetate. Phase-transfer catalyst stays in the aqueous layer during extraction.

## Claims

1. An aldehyde condensation process comprising contacting or combining a first aldehyde selected from the aldehydes containing at least one α-hydrogen atom having the formula R₁CHO, wherein R₁ is selected from alkyl having one to twelve carbon atoms, and aralkyl having seven to fourteen carbon atoms, with a second different aldehyde selected from aldehydes having the formula R₂CHO, wherein R₂ is selected from H, alkyl having one to twelve carbon atoms, and aralkyl having seven to fourteen carbon atoms, and with a catalytic amount of only a catalyst of Formula (1): 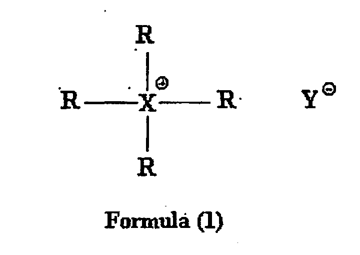 wherein R = same or different alkyl or aralkyl group, x = nitrogen or phosphorous atom, y = OH counter ion.

2. A process according to Claim 1, wherein aldehydes R₁CHO or R₂CHO may be taken in 5-10 % excess with respect to each other.

3. A process according to Claim 1 or 2, wherein the reaction product mixtures of the invention will comprise or contain one of (A) the aldol of the aldehyde condensed, or of the aldehydes cross condensed; (B) the unsaturated aldehyde derived by dehydration of said aldol; or (C) a mixture of (A) and (B).

4. A process according to any of Claims 1 to 3, for the synthesis of hydroxypivaldehyde comprising reacting isobutyraldehyde and formaldehyde/or paraformaldehyde, or trioxane and catalytic amount of the phase-transfer catalyst of Formula (I), such as benzyltrimethylammonium hydroxide or benzyltriethylammonium hydroxide.

5. A process according to any of the preceding Claims, wherein the molar ratio of phase-transfer catalyst to aldehyde R₁CHO is 0.5.0 % to 10 % equivalent, most preferably 4 mole % equivalent.

6. A process according to any of the preceding Claims, wherein the reaction temperature is 18 - 23°C, most preferably 20°C.

7. A process according to any of the preceding Claims, wherein the reaction time is 10-90 minutes, most preferably 30 - 90 minutes.

8. A process according to any of the preceding Claims, wherein the aldol product can be isolated by extraction with a suitable solvent while allowing the phase-transfer catalyst in the aqueous layer.

9. A process according to any of the preceding Claims, wherein the reaction product mixture can be directly subjected to catalytic hydrogenation for making polyvalent alcohol such as neopentyl glycol.

10. A process according to any of the preceding Claims, wherein the phase-transfer catalyst may be added to the reaction mixture in the solid form (when available) or in the form of an aqueous or an alcoholic solution.

## Patentansprüche

1. Aldehydkondensationsverfahren, welches umfaßt ein Kontaktieren oder Kombinieren eines ersten Aldehyds, welches ausgewählt wird aus den Aldehyden mit der Formel R₁CHO, welche wenigstens ein α-Wasserstoffatom enthalten, wobei R₁ ausgewählt wird aus Alkyl mit einem mit zwölf Kohlenstoffatomen und Aralkyl mit sieben bis vierzehn Kohlenstoffatomen, mit einem zweiten unterschiedlichen Aldehyd, welches ausgewählt wird aus Aldehyden mit der Formel R₂CHO, wobei R₂ ausgewählt wird aus H, Alkyl mit einem bis zwölf Kohlenstoffatomen und Aralkyl mit sieben bis vierzehn Kohlenstoffatomen, und mit einer katalytischen Menge lediglich eines Katalysators der Formel (1): 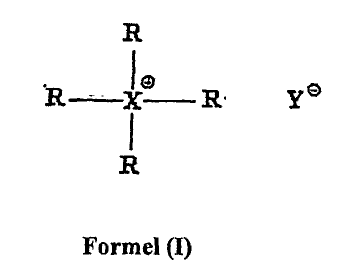 worin R = gleiche oder unterschiedliche Alkyl- oder Aralkylgruppen, x = Stickstoffoder Phosphoratom, y = OH-Gegenion ist.

2. Verfahren nach Anspruch 1, **dadurch gekennzeichnet, daß** die Aldehyde R₁CHO oder R₂CHO in einem 5-10%igen Überschuß in Bezug zueinander vorliegen.

3. Verfahren nach Anspruch 1 oder 2, **dadurch gekennzeichnet, daß** die Reaktionsproduktmischungen der Erfindung umfassen oder beinhalten entweder (A) das Aldol des kondensierten Aldehyds oder des cross-kondensierten Aldehyds; (B) den ungesättigten Aldehyd, welcher durch Dehydratisierung des Aldols erhalten wird; oder (C) eine Mischung aus (A) und (B).

4. Verfahren nach einem der Ansprüche 1 bis 3 für die Synthese von Hydroxypivaldehyd, welches umfaßt ein Umsetzen von Isobutyraldehyd und Formaldehyd oder Paraformaldehyd oder Trioxan und katalytischen Mengen des Phasentransferkatalysators der Formel (I), wie Benzyltrimethylammoniumhydroxid oder Benzyltriethylammoniumhydroxid.

5. Verfahren nach einem der vorangehenden Ansprüche, **dadurch gekennzeichnet, daß** das molare Verhältnis von Phasentransferkatalysator zu Aldehyd R₁CHO 0,5 % bis 10 % ist, am bevorzugtesten gleich 4 Mol-%.

6. Verfahren nach einem der vorangehenden Ansprüche, **dadurch gekennzeichnet, daß** die Reaktionstemperatur 18 bis 23°C ist, am bevorzugtesten 20°C.

7. Verfahren nach einem der vorangehenden Ansprüche, **dadurch gekennzeichnet, daß** die Reaktionszeit 10 bis 90 Minuten beträgt, am bevorzugtesten 30 bis 90 Minuten.

8. Verfahren nach einem der vorangehenden Ansprüche, **dadurch gekennzeichnet, daß** das Aldolprodukt durch Extraktion mit einem geeigneten Lösungsmittel isoliert werden kann, während der Phasentransferkatalysator in der wäßrigen Schicht vorliegt.

9. Verfahren nach einem der vorangehenden Ansprüche, **dadurch gekennzeichnet, daß** die Reaktionsproduktmischung unmittelbar einer katalytischen Hydrierung zur Herstellung mehrwertiger Alkohole, wie Neopentylglykol, unterworfen werden kann.

10. Verfahren nach einem der vorangehenden Ansprüche, **dadurch gekennzeichnet, daß** der Phasentransferkatalysator zu der Reaktionsmischung in der festen Form (wenn verfügbar) oder in der Form einer wäßrigen oder einer alkoholischen Lösung zugefügt werden kann.

## Revendications

1. Procédé de condensation d'aldéhydes consistant à mettre en contact ou à combiner un premier aldéhyde choisi parmi les aldéhydes contenant au moins un atome d'hydrogène α possédant la formule R₁CHO, dans laquelle R₁ est choisi parmi un groupe alkyle possédant un à douze atomes de carbone, et un groupe aralkyle possédant sept à quatorze atomes de carbone, avec un second aldéhyde différent choisi parmi les aldéhydes possédant la formule R₂CHO, dans laquelle R₂ est choisi parmi H, un groupe alkyle possédant un à douze atomes de carbone, et aralkyle possédant sept à quatorze atomes de carbone, et avec une quantité catalytique d'un seul catalyseur de formule (I) : 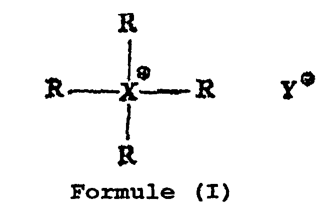 dans laquelle R = groupe alkyle ou aralkyle identique ou différent, x = atome d'azote ou de phosphore, y = contre-ion OH.

2. Procédé selon la revendication 1, dans lequel les aldéhydes R₁CHO ou R₂CHO peuvent être pris dans un excès de 5 à 10 % les uns par rapport aux autres.

3. Procédé selon la revendication 1 ou 2, dans lequel les mélanges de produits de réaction de l'invention vont comprendre ou contenir l'un parmi (A) l'aldol de l'aldéhyde condensé, ou des aldéhydes croisés condensés ; (B) l'aldéhyde insaturé dérivé par déshydratation dudit aldol, ou (C) un mélange de (A) et (B).

4. Procédé selon l'une quelconque des revendications 1 à 3, destiné à la synthèse d'hydroxypivaldéhyde consistant à faire réagir de l'isobutyraldéhyde et du formaldéhyde ou du paraformaldéhyde, ou du trioxanne et une quantité catalytique du catalyseur de transfert de phase de formule (I), tel que l'hydroxyde de benzyltriméthylammonium ou l'hydroxyde de benzyltriéthylammonium.

5. Procédé selon l'une quelconque des revendications précédentes, dans lequel le rapport molaire du catalyseur de transfert de phase à l'aldéhyde R₁CHO est équivalent à 0,50 % à 10 %, de manière tout particulièrement préférée équivalent à 4 % en moles.

6. Procédé selon l'une quelconque des revendications précédentes, dans lequel la température de réaction est de 18°C à 23°C, de manière tout particulièrement préférée de 20°C.

7. Procédé selon l'une quelconque des revendications précédentes, dans lequel la durée de réaction va de 10 à 90 minutes, de manière tout particulièrement préférée de 30 à 90 minutes.

8. Procédé selon l'une quelconque des revendications précédentes, dans lequel le produit d'aldol peut être isolé par extraction avec un solvant approprié tout en laissant le catalyseur de transfert de phase dans une couche aqueuse.

9. Procédé selon l'une quelconque des revendications précédentes, dans lequel on peut soumettre directement le mélange de produits de réaction à une hydrogénation catalytique pour réaliser un alcool polyvalent tel que le néopentylglycol.

10. Procédé selon l'une quelconque des revendications précédentes, dans lequel on peut ajouter le catalyseur de transfert de phase au mélange réactionnel sous la forme solide (quand disponible) ou sous la forme d'une solution aqueuse ou d'une solution alcoolique.
